(19) Europäisches Patentamt
European Patent Office
Office européen des brevets

(11) **EP 2 890 755 B1**

(12) **EUROPEAN PATENT SPECIFICATION**

(45) Date of publication and mention
of the grant of the patent:
**10.10.2018   Bulletin 2018/41**

(51) Int Cl.:
***C09J 131/02*** *(2006.01)*  ***C09J 131/04*** *(2006.01)*
***C09J 11/06*** *(2006.01)*  ***C09J 123/22*** *(2006.01)*

(21) Application number: **13833724.1**

(86) International application number:
**PCT/US2013/057239**

(22) Date of filing: **29.08.2013**

(87) International publication number:
**WO 2014/036230 (06.03.2014 Gazette 2014/10)**

(54) **PRESSURE SENSITIVE ADHESIVE COMPOSITIONS CONTAINING PLANT AND ANIMAL BUTTERS**

DRUCKEMPFINDLICHE KLEBSTOFFZUSAMMENSETZUNGEN MIT BUTTER PFLANZLICHEN UND TIERISCHEN URSPRUNGS

COMPOSITIONS ADHÉSIVES SENSIBLES À LA PRESSION CONTENANT DES BEURRES ANIMAUX ET VÉGÉTAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.08.2012   US 201261694738 P**
**20.08.2013   US 201313971384**

(43) Date of publication of application:
**08.07.2015   Bulletin 2015/28**

(73) Proprietor: **Euromed Inc.**
**Orangeburg, NY 10962 (US)**

(72) Inventors:
• **DONG, Ying**
**Pomona, New York 10970 (US)**
• **RAMJIT, Ravi**
**Dix Hill, New York 11746 (US)**
• **GANGOLLI, Sridhar**
**Park Ridge, New Jersey 07656 (US)**

(74) Representative: **Haseltine Lake LLP**
**Redcliff Quay**
**120 Redcliff Street**
**Bristol BS1 6HU (GB)**

(56) References cited:
**US-A1- 2004 241 246     US-A1- 2004 265 268**
**US-A1- 2005 130 871     US-A1- 2007 026 056**
**US-A1- 2007 190 124     US-A1- 2007 258 935**
**US-A1- 2011 118 363**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of the invention

The present invention relates to a pressure sensitive, moldable adhesive dressing composition suitable for various medical device applications,particularly ostomy care applications to prevent leakage around the stoma which can lead to damage of healthy neighboring skin. Furthermore, the adhesive dressing composition can be molded to maximize coverage and protection of various abdominal anatomies, sealing the area between the skin and ostomy appliance. In particular, the present invention relates to a pressure sensitive adhesive composition comprising polyethylene copolymers, polystyrene copolymers, polyisobutylene copolymers and various butters including, but not limited to, shea butter, avocado butter, cocoa butter, aloe butter and animal butters such as butter derived from bovine milk.

### Background of the Invention

Pressure sensitive adhesives for ostomy appliances and wound dressing have been used for many years. For example, hydrocolloid adhesives containing hydrophilic absorbents, which absorb fluids and moisture, are one type of unique pressure sensitive adhesives in medical devices. Patients with surgery for a variety of diseases related to the ostomy or urinary tract, typically cancer, have to wear the collecting device permanently. Thus, adhesion of a collecting device to body skin must securely stay in place and have enough ability to absorb the fluids and moisture without detaching. When removing the adhesive, the adhesive should be cohesive enough to remove without leaving residues on the skin, while at same time avoiding damage to the skin..

Conventional pressure sensitive adhesives embody these functions, such as adhering to skin and providing a barrier to leakage., There are, however, few products which consider skincare, skin protection, or even promoting skin health.

Thus, there is a need for pressure sensitive adhesives with skin care properties in mind. Specifically, developing adhesives that have the properties to protect and prevent skin disease or even provide skincare functions while still having a good moisture absorption and fixation is particularly important when a medical device has to be worn on the skin for a long time to protect the skin from corrosive fluids discharged by the stoma and wound. For the patients who have to wear collecting device for a long term, the most common problems are peristomal skin complications. These complications includes skin excoriation or irritation from faecal output; mechanical irritation related to the removal of adhesives appliances; allergies or sensitivities to chemicals or polymers; folliculitis (infected hair follicles); and monillia/candida albicans which is caused by yeast infection.

Faecal enzymes, moisture and chemical/biological irritants (Berg, 1998; Buckingham and Berg, 1986) are three major contributing factors for peritomal skin complications. Faecal enzymes, in particular; proteases and lipases in stomal output have been reported to be one of the major factors in breaking down skin integrity causing irritation (Buckingham and Berg, 1986). About 45% of patients will suffer from a peristomal skin disorder (Herlufsen et al., 2006). Thus, prevention and management of peristomal skin complications are critical components of ostomy care (Rolstad et al., 2004).

Generally, almost all of skin adhesive agents comprise a polymer or copolymer in which hydrocolloids, such as water absorbing, swollen particles are dispersed. To soften the polymer or copolymer, plasticizers, such as mineral oil, or poly (propylene oxide) have to be added.

US patent 6437038 describes a pressure sensitive adhesive composition comprising 15-60 butyl rubber, 15-60% one or more tackifying resins, 1-10% a tackifying liquid constituent, e.g. paraffin oil, and 20-60% of one or more hydrocolloids.

US patent 4551490 describing adhesives containing SIS/SI, PIB/butyl rubber, tackifier, mineral oil and hydrocolloids.

US patent 6933342 describes a formulation comprising a triblock copolymer, a terpene resin, a liquid and EVA polymer wherein the EVA polymer preferably possess a vinyl acetate content of more than 50% by weight.

The use of hydrocolloid pressure sensitive dressings and an ostomy seal has been described in, for example, US patents 6685683 disclose skin barrier product comprising starch for modifying of the rheological properties of a zone of an adhesive flexible skin barrier product layer for an ostomy appliance.

US patent 3712304 and 3799166 describes an ostomy seal made from starch and gelatinized starch cross-linked with glyoxal.

US patent 5633010 discloses an invention of adhesive composition comprising a hydrophobic unsaturated aliphatic homopolymer cross-linked by a dose of radiation, a compatible tackifier, and at least one hydrocolloid absorbent. These adhesive compositions exhibit consistently high wet integrity, and thus provide dressings that maintain their form and impart a minimum amount of hydrocolloid residue.

US patent 2011/0251300 describes a soft shapeable adhesive paste. The composition can be used for smoothing out irregular skin area for subsequent secure attachment and has improved erosion resistance, without compromising the moisture absorption, ease of removal or introducing other possible adverse effects.

US patent 2010/0204632 disclosure permeable pressure sensitive adhesive by using polar oils and polar polyethylene copolymers. These adhesives have a very high moisture vapor transmission rate, which make them breathable and very skin friendly.

US patent 2011/0251542 describes polyisobutylene can be used as a tackifying agent which lowers the amount of cell stripping compared to resins.

WO 2009/006901 describes polyisobutylene with low glass transition temperature can be added to the polar adhesives, even though apolar in nature.

US patent 2011/0118363 describes using polar oil or fat including (a) at least one triglyceride, and/ or at least one fatty acid of the formula $R-CO_2H$, wherein R is C3 to C30 alkyl group; and (ii) at least one homopolymer, and /or copolymer. This adhesive composition carries unique features of skin friendliness, e.g. hypoallergenicity, painless removal, repositionability, and ability to initiate natural antimicrobial activities.

None of the adhesives described above contain butters derived from plants and animals, such as shea butter.

US 2011/0118363 proposes an adhesive composition applicable to skin and US 2004/0241246 proposes an adhesive paste applicable to skin.

Generally, mineral oil is widely used in adhesive formulations; however shea butter which is typically used in cosmetic applications, has not been reported in wound care adhesive or ostomy seal applications. By substituting shea butter for mineral oil, this skin friendly, biocompatible, multifunctional component gives more advantage and provides more and unexpected benefits for skin than biologically inert oils such as mineral oil.

Specifically, Applicants have found that butters derived from plants and animals are useful in reducing the skin irritation experienced by ostomy wearers. Useful butters include, but are not limited to, shea, cocoa, orange peel, aloe and animal butters derived from animal milk such as cow milk. By way of example, shea butter contains many fatty acids: e.g. oleic acid (40-60%), stearic acid (20-50%), linoleic acid (3-11%), palmitic acid (2-9%), linolenic acid(<1%) and arachidic acid(<1%). These carboxylic groups provide an excellent skin adhesive, and at same time will buffer skin surface pH at a natural level of pH 5.5 or below to 7 and thus reduce enzyme activity.

**Summary of the invention**

The present invention relates to a pressure sensitive adhesive composition comprising:

a. 2 to 45 % by weight of a homopolymer or copolymer selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide, polystyrene copolymers and mixtures thereof;

b. 1 to 10 % by weight of shea butter;

c. 15- 45 % by weight of a tackifier selected from the group consisting of natural rosin, modified rosin, glycerol ester of natural rosin, glycerol-ester of modified rosin, pentaerythritol ester of natural rosin, pentaerythritol ester of modified rosin, phenolic modified terpene resin, aliphatic petroleum hydrocarbon resin, cycloaliphatic resin and mixtures thereof.

**Brief description of the drawings**

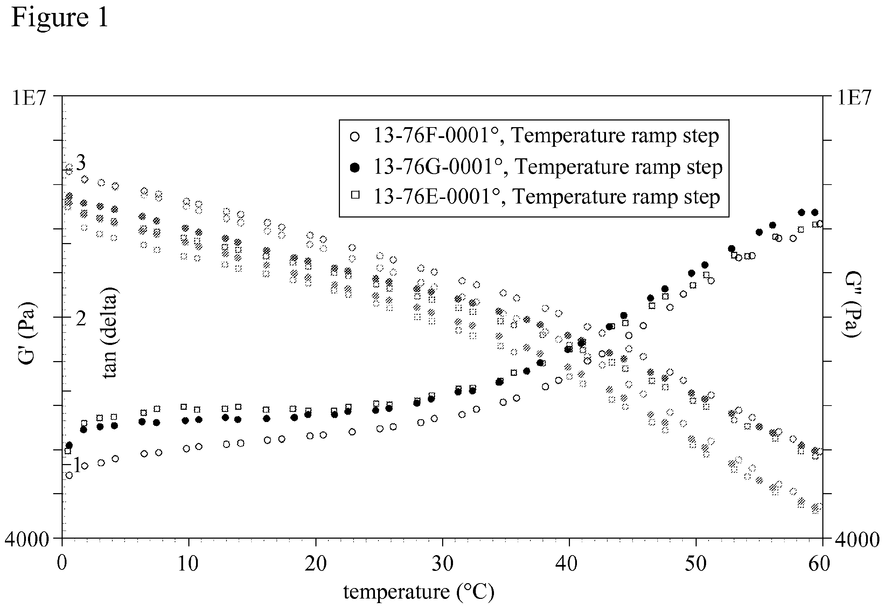

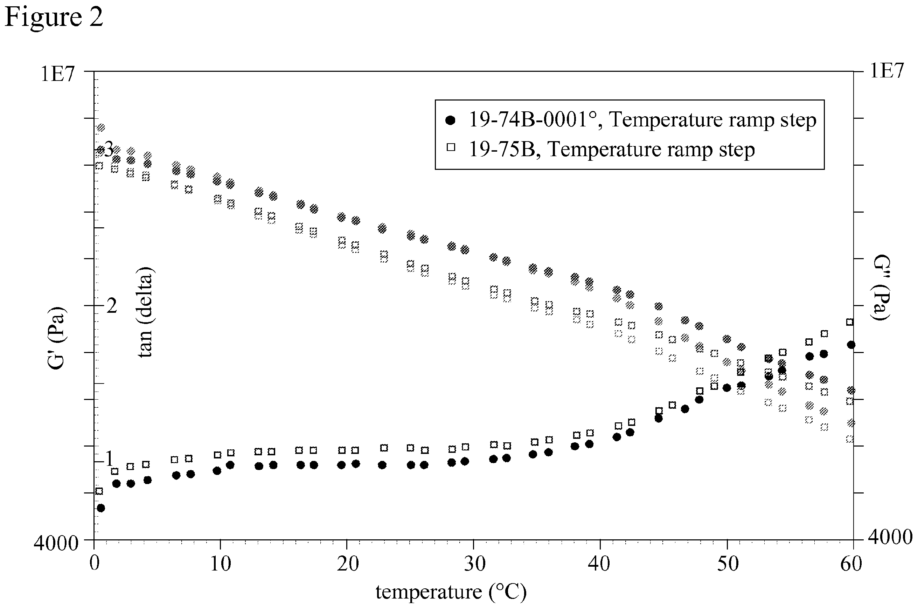

Properties of the invention are shown in more detail in Fig.1 and 2, which show the shear modulus G*[Pa] vs. temperature and loss tangent (tan $\delta$) curve vs. the temperature profiles of the compositions according to the invention.

Fig. 1 shows the results of elastic modulus G* and tangent (tan $\delta$) curves for the samples 13-76E, 13-76F and 13-76G from 0°C to 60°C.

Of all three samples have the same base polymer Kraton 1114 PT, styrenic block copolymer and other ingredients, only slightly amount % difference. Sample 13-76F contains no shea butter, sample 13-76G contains 1.62% and sample 13-76E has 1.81% of total weight. It shows a significant difference of elastic modulus change when the shea butter increases from 0% to 1.81%, G* from 2.56 x $10^5$ to1.42 x$10^5$ Pa at 32.4°C, that is, a 44% change G* value in comparison of sample 13-76F with 13-76E. This indicates that even a small amount of shea butter will play a role of softening the adhesive mixture. Sample 13-76G has a similar result with G* decreasing to 34% when the amount of shea butter increases from 0% to 1.62%.

Fig. 2 shows the results of elastic modulus G* and tangent (tan $\delta$) curves for the samples 19-74B and 19-75B from 0°C to 60°C.

Sample 19-74B and 19-75B base polymer are Elvax 260, polyethylene vinyl acetate block copolymer, the tackfier is Arkon P115. The other ingredients are the same as above three samples. Sample 19-75B has 0.45% more shea butter than sample 19-74B, however, the elastic modulus are changes 45% from $4.1 \times 10^5$ to $2.2 \times 10^5$ Pa at 32.7°C. This also demonstrates that shea butter as rheology modifier and softening agent is a critical material to control the adhesive properties in the designed elastic modulus range.

## Detailed description of the invention

The present invention relates to a pressure sensitive adhesive composition comprising:

a. 2 to 45 % by weight of a homopolymer or copolymer selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide, polystyrene copolymers and mixtures thereof;

b. 1 to 10 % by weight of shea butter;

c. 15- 45 % by weight of a tackifier selected from the group consisting of natural rosin, modified rosin, glycerol ester of natural rosin, glycerol-ester of modified rosin, pentaerythritol ester of natural rosin, pentaerythritol ester of modified rosin, phenolic modified terpene resin, aliphatic petroleum hydrocarbon resin, cycloaliphatic resin and mixtures thereof.

It has been found that the pressure sensitive adhesive compositions with butters derived from plants and animals exhibit unique features that can be used for a variety of applications for medical devices, such as skin adhesives, wound dressings, and ostomy seals. Features of good adhesion to skin, high water absorption, easy shapeable and easy moldable, skin friendliness are obtained by combined with other copolymer such as polyethylene copolymer, poly(isobutylene) and optionally other ingredients, e.g. one or more tackifier resins, and one or more hydrocolloids. For example, Shea butter, a complex mixture of fat and vitamins A and E, on the other hand, will help moisturize skin, protect skin from damage, and in some case even slow down or cure skin diseases. Shea butter also plays a role as rheology modifier. These multiple functions lead to a very special character of skin friendliness, e.g. protection, prevention, healing, prepositional and easy of removal of pressure sensitive adhesives for skin fixation, wound dressing and ostomy seal applications.

By introducing shea butter, the stability issues due to phase separation or migration are avoided. The properties of shea butter as an amphiphile allow it to combine polar and non-polar materials. Dynamic mechanical analysis shows uniform curves of (G*) and tan ($\delta$) with combined rheological expressions of both polar and non-polar parts.

By addition of shea butter, a rheology modifier and plasticizer for ostomy and other medical devices, it is possible to achieve a softer paste that is easier to shape. Shea butter in ostomy paste and medical device fixation applications has not been reported. Shea butter will buffer the skin in the proper pH range and reduce enzyme activity, to prevent both skin diseases to happen and the paste to break down either. Said butter comprises 1-10% of the final formulation.

The pressure sensitive adhesive composition of the present invention differs from the compositions disclosed in U.S. Pat.Nos.2011/0251542A1 and2011/0251300A1 in that they do not include shea butter.

The pressure sensitive adhesive composition of the present invention differs from the composition of disclosed from in U.S. Pat. Nos. 2010/0204632A1 in that they comprise the polyethylene copolymer has a melt flow index below 2 g/10min and in that the polyethylene copolymer has a melt flow index above 2 g/10min.

It is also found that shea butter as softening agent and moisturizer leads to a comfortable texture and feel on the part of the user.

In this present invention, the design approach is directed toward obtaining an adhesive to be moldable, easily shaped, moisture absorbing skin barrier, at same time also can be stretched, compressed or molded to fit the exact shape and size as required, which means adhesive should not have memorable property in order to keep the designed shape. Thus the G' has to keep certain high level while still keep the properties such as tacky and moldable. To achieve the above required properties, adhesive G' and tan $\delta$ in designed range are very critical. By addition of shea butter to the adhesive composition yielded surprisingly desired results. By adjusting the amount of shea butter, both the rheology G' and tan delta can be obtained in the designed range.

In one embodiment of the present invention, the adhesive composition comprises shea butter with vitamins E and A and at least one homopolymer, and /or block copolymer. The homopolymer includes, but is not limited to isobutylene, polyethylene, polypropylene, polybutylene, polystyrene.

A suitable block copolymer contains at least two monomers which have very different glass transition temperatures so that they are immiscible in each other and phase separated at room temperature. Examples of such copolymers include, but are not limited to, styrene isoprene styrene, styrene butadiene styrene, styrene propylene styrene, and ethylene vinyl acetate and ethylene acrylate.

The primary polymers used in the adhesive composition are ethylene copolymers, the polymer should contain considerable amount of a polar component to soften and get water permeability. Preferably, the ethylene parts of the copolymer can form crystalline areas that ensure the cohesive strength of the adhesive. Said primary polymers comprise 2-45% of the final formulation.

In one embodiment of the invention, the polar polyethylene copolymer is selected from the group consisting of ethylene vinyl acetate, ethylene vinyl acetate carbon monoxide, ethylene butyl acetate, ethylene vinyl alcohol, ethylene butyl acrylate, ethylene butyl acrylate carbon monoxide and combinations thereof.

The polar polyethylene copolymer is preferably ethylene vinyl acetate with 18-30% weight of vinyl acetate; especially prefer 28% of vinyl acetate.

In one of embodiment, the present invention, the adhesive composition comprises shea butter with vitamin E and A and at least one homopolymer, and /or copolymer, and a tackifier.

The tackifiers applicable to this invention adhesive include, but not limited to, natural rosin, modified rosin, glycerol ester of natural rosin, glycerol-ester of modified rosin, pentaerythritol ester of natural rosin, pentaerythritol ester of modified rosin, phenolic modified terpene resin, aliphatic petroleum hydrocarbon resin, and cycloaliphatic resin.

Tackifier can used to control the tack of the adhesive and reduce moduli and increase glass transition temperature.

Said tackifiers comprise 15-45% of the final formulation, more preferably from 20-35%.

In one of embodiment, the present invention, the adhesive composition comprising plasticizer selected from the group of mineral oil, citrate oil, paraffin oil, phthalic acid esters, adipic acid esters and liquid or solid resin.

In one embodiment of the present invention, the adhesive composition further comprises a polyethylene wax.

In another embodiment of the invention, the composition of the ostomy seals or skin fixation device further comprises other ingredients selected from a group of antioxidants, stabilizers, fillers, pigments, flow modifiers, and active ingredients such as drugs.

In one of embodiment, the present invention the adhesive composition comprises shea butter with vitamin E and A, and at least one homopolymer, and /or copolymer, a tackifier and a hydrophilic liquid absorber or gel-thickener.

Suitable hydrophilic liquid absorbers or gel-thickeners are selected from naturally occurring hydrocolloids, semisynthetic hydrocolloids and synthetic hydrocolloids.

More particularly, the hydrocolloids are preferably selected from guar gum, locust bean gum, pectin, alginates, gelatin, xanthan and /or gum karaya; cellulose derivatives such as sodium carboxymethylcellulose, methylcellulose and hydroxypropylmethylcellulose and /or sodium starch glycolate and /or polyvinyl alcohol and/or polyethylene glycol.

It is preferred to use a combination of two or more hydrocolloids. It is especially preferred to use combination of guar gum and sodium carboxymethylcellulose as the hydrocolloid component.

In one embodiment of the invention, the content of hydrocolloid is 20-60% of the total composition, more preferably from 30-45%.

According to one embodiment of the invention, the final paste has an elastic modulus of less than 1000000 Pa, preferable less than 300000 Pa at 1 Hz (1% deformation, 32°C).

According to one of the inventions, the final paste has a tan ($\delta$) above 0.8, preferably above 1.0 at 1Hz (1% deformation, 32°C).

The following non limiting examples illustrate the practice of the present invention.

**Experimental**

Materials and methods

Materials

Table 1

| Description | Manufacturer | Property |
|---|---|---|
| Oppanol 10 | BASF | Base polymer |
| Elvaloy® AC 1125 | Dupont | Elastomer |
| Liposorb TS | Lipo Chemical | Rheology modifier |
| Wingtack 95 Flake | Eastman | Tackifier |
| Guar NT 3500F | TIC Gums Inc | Water absorbent |

(continued)

| Description | Manufacturer | Property |
|---|---|---|
| Carboxymethylcellulose F1-4000XX | Amtex | Water absorbent |
| Microcrystalline Cellulose (MCC) | FMC Bio polymer | Filler |
| LipoButter, Refined Shea (non-organic) | Lipo Chemical | Rheology modifier |

PIB: Polyisobutylene available under trademark oppanol from BASF chemical company
Poly ethylene vinyl acetate and poly ethylene acrylate from Dupont chemical company.
Kraton D1114PT: Styrene-isoprene-styrene copolymer from Kraton company.
Wingtack 95: hydrocarbon resins from Cray valley.
Amtex CMC: Sodium carboxymethylcellulose from Amtex chemicals
GuarNT3500F powder: from TIC Gums, Inc.
MCC: Microcrystalline Cellulose from FMC Bio polymer with trade name PH-200NF.
Lipobutter Refined Shea: Refined shea butter from Lipo chemicals.
Liposorb® TS: Sorbitan tristearate from Lipo chemicals.

Elvax 260, Elvaloy and Kraton polymer provides the cohesive strength. PIB provides the main foundation of the moldable adhesive. Shea butter and Liposorb TS serves as rheology modifiers and skin friendly moisturizing agents. MCC plays the role as filler. Amtex CMC and guar gum absorb fluids. Wingtack 95 helps adhesive and initial tack property.

Example 1-2

Preparation of adhesive materials according to the invention having the composition stated in Table 2 below:

Table 2

| Description | Mix EL1108161 % | Mix EL1108241 % |
|---|---|---|
| Elvaloy® AC 1125 | 5.44 | 5.43 |
| LipoButter, Refined Shea | 2.44 | 2.43 |
| Oppanol 10 | 33.52 | 33.49 |
| Microcrystalline Cellulose (MCC) | 5.23 | 5.30 |
| Wingtack 95 Flake | 17.38 | 17.35 |
| Amtex CMC F1-4000XX | 22.12 | 22.70 |
| Liposorb TS | 6.28 | 6.32 |
| Guar NT 3500F | 7.61 | 6.97 |
| Total | 100.00 | 100.00 |

Method of mixing

Elvaloy® AC 1125 was added in to the Z-mixer at 165°C and kneaded by the mixing machine for 15 minutes. Shea butter was added and mixing was continued at 165 °C for 15-30 minutes, after which Oppanol 10 was added until the blend was homogenous. Then MCC was added until there were no polymer chunks and whole mixture was homogenous. To the mixture Wingtack 95 was added and kneaded for 15 min, and then following Amtex CMC, Liposorb TS were added in order at intervals of a few minutes without continuing to heat the mixer. When the temperature cooled to 100°C or below, Guar gum was added until a homogeneous mixture was formed.

Examples 3-5

Following similar procedure disclosed in the Example 1-2 adhesive composition according to the invention having the compositions stated in Table 3 were prepared.

Table 3

| Component | Mix13- 76E % | Mix13- 76F % | Mix 13-76G % |
|---|---|---|---|
| Kraton1114PT | 4.02 | 4.16 | 4.25 |
| Oppanol 10 | 28.79 | 31.16 | 31.01 |
| Wingtack95 | 17.79 | 18.19 | 17.68 |
| Amtex CMC | 23.84 | 25.85 | 25.94 |
| Guar NT 3500F | 8.83 | 7.28 | 7.26 |
| MCC | 4.72 | 5.22 | 5.02 |
| Shea butter | 1.81 | 0.00 | 1.62 |
| Liposorb TS | 6.18 | 8.14 | 7.21 |
| Total | 100.00 | 100.00 | 100.00 |

The examples in Table 3 comprise mixtures different from examples 1-2. Kraton 1114PT, Styrenic block copolymer, replaced Elvaloy® AC 1125 as cohesive strength material.

Examples 6-7

Following similar procedure disclosed in the Example 1-2 adhesive composition according to the invention having the compositions stated in Table 4 were prepared.

Table 4

| Component | Mix 19-74B | Mix 19-75B % |
|---|---|---|
| Elvax260 | 8.34 | 8.33 |
| Oppanol 10 | 29.41 | 29.30 |
| Arkon p115 | 17.90 | 18.03 |
| MCC | 5.56 | 5.54 |
| Amtex CMC | 22.60 | 22.53 |
| Shea butter | 3.66 | 4.11 |
| Liposorb TS | 5.57 | 5.21 |
| Guar gum | 6.96 | 6.94 |
| Component | Mix 19-74B % | Mix 19-75B % |
|  | 100.00 | 100.00 |

The Table 4 the samples differ from examples 1-2 in that Elvax 260, polyethylene vinyl acetate block copolymer, replaced Elvaloy® AC 1125 as cohesive strength material.

Examples 8-9

Water absorption measurement

Sample prepare method

The adhesive was pressed between two pieces of silicone paper and PU-film paper by compressor mold at 85°C and 10000lb force with thickness of 1 mm, the sample covered with the release liner with thickness 0.18 mm and PU-film with thickness 0.02 mm. A sample of 1' diameter was then punched out while the double sticky tape was adhering on the PU-film.

Method of determination of water absorption

Water absorption measurement

The sample with PU-film and double sticky film was weighed and placed in container with 0.9% saline at 37°C oven. After 2hours, 4 hours, 6 hours, 18 hours and 24 hours, the sample was removed from the container, the water was shaken off, and the sample was weighed again. The increase of weight was recorded as the water absorption. The water absorption was calculated as

$$\text{Water absorption after 2 hours} = M(2 \text{ hours}) - M(\text{start})/5.026[g/cm^2]$$

Table 5

| Water Absorption (g /10 cm$^2$) | | | | | |
|---|---|---|---|---|---|
| Sample # | 2hrs | 4hrs | 6hrs | 18hrs | 24hrs |
| EL1108161 | 2.84 | 4.16 | 5.15 | 9.56 | 9.39 |
| EL1108241 | 3.17 | 4.46 | 5.09 | 10.18 | 8.62 |

Samples EL1108161 and EL1108241 showed a similar level of water absorption at 2h, 4h, 6h 18h and 24h. It shows that a small percentage of change of shea butter in the adhesives composition did not play the big role in water absorbance as rheology relatively. Water absorbance reaches the peak at 18 hour for both of the samples.

Examples 10-12

Table 6

| Water Absorption (g /10 cm2) | | | |
|---|---|---|---|
| Sample # | 2hrs | 4hrs | 24hrs |
| 13-76E | 3.63 | 4.75 | 10.09 |
| 13-76F | 3.27 | 4.57 | 9.62 |
| 13-76G | 3.63 | 4.61 | 8.58 |

Samples 13-73E, 13-76F and 13-76G also showed the similar level of water absorption at 2h, 4h, 6h 18h and 24h. It gives the same conclusion even with different the base polymer that small amount of change of shea butter in the adhesives composition has not significant affect in water absorbance as rheology relatively.

Example 13-14

Table 7

| Water Absorption (g/10 cm$^2$) | | | | | |
|---|---|---|---|---|---|
| Sample # | 2hrs | 4hrs | 6hrs | 18hrs | 24hrs |
| 19-74B | 2.40 | 4.39 | 5.71 | 11.66 | 12.86 |
| 19-75B | 2.46 | 4.07 | 5.42 | 11.71 | 13.21 |

Samples 19-74B and 19-75B have a higher percentage of base polymers than examples in Tables 5 and 6. Thus the initial water absorbance is lower than in the polymers in Tables 5 and 6, however, the water absorbance keeps increasing even after 18 hour, and is highest at 24 hours of all above samples. The small percentage increase in shea butter in the adhesive composition has no significant effect on water absorbance, although it has a large effect on the elastic modulus (see Figures 1 and 2). Moreover, the cohesive strength is maintained due to the high percentage of the homopolymer of copolymer.

Determination of probe tack

Probe tack test were carried out with probe tack instrument of EZlab Compatible from ChemInstruments;

The samples were prepared in the same way as water absorbance except with double sticky tape on one side.

The test results show a little slightly higher probe tack than for the existing commercial product available under the trademark Eakin cohesive seal.

Example 15

Table 8

| Sample # | 13-76E | 13-76F | 13-76G |
|---|---|---|---|
| Probe Tack(g) | 199 | 126 | 198 |
| Shea butter, % | 1.81 | 0 | 1.62 |

The example 15 illustrates that a small amount of shea butter increases the probe tack.

Example 16

Table 9

| Sample # | EL1108161 | EL1108241 |
|---|---|---|
| Probe Tack(g) | 182 | 188 |
| Shea butter, % | 2.44 | 2.43 |

Example 16 shows the probe tack of two formulations with shea butter.

Example 17

Table 10

| Sample # | 19-74B | 19-75B |
|---|---|---|
| Probe Tack(g) | 65 | 97 |
| Shea butter, % | 3.66 | 4.11 |

The results of Table 10 show that a small increase in shea butter concentration raises the probe tack slightly. At same time, the base polymer percentage in the formulation causes the changes of both mechanical and physical properties. Probe tack and water absorbance decrease as long polymer concentration increases.

Example 18

Test of rheological properties

Test of the elastic properties as measured by Dynamic Mechanical Analysis of composition of examples 13-76E, 13-76 F, and 13-76 in Fig 1, 19-74B and 19-785B in
Fig 2. of the present application have been carried out.

Dynamic Mechanic Analysis (DMA)

The analysis was carried out by using a TA2000 EX apparatus.
Total viscoelastic modulus (*G) and tan $\delta$ were determined at a frequency sweep 1% from temperature 0°C to 60°C.
The three samples 13-76E, 13-76 F and 13-76 had similar formulations except sample 13-76F without shea butter. The samples 19-74B and 19-78B base polymer are polyethylene vinyl acetate copolymer, while the other above three samples base polymer contain Kraton 1114PT, a styrene-isoprene-styrene copolymer.

**Claims**

1.   A pressure sensitive adhesive composition comprising:

   a. 2 to 45 % by weight of a homopolymer or copolymer selected from the group consisting of polyisobutylene, ethylene vinyl acetate, ethylene vinyl acetate carbon monoxide, ethylene butyl acetate, ethylene vinyl alcohol, ethylene butyl acrylate, ethylene butyl acrylate carbon monoxide, polystyrene copolymers and mixtures thereof;
   b. 1 to 10 % by weight of shea butter;
   c. 15- 45 % by weight of a tackifier selected from the group consisting of natural rosin, modified rosin, glycerol ester of natural rosin, glycerol-ester of modified rosin, pentaerythritol ester of natural rosin, pentaerythritol ester of modified rosin, phenolic modified terpene resin, aliphatic petroleum hydrocarbon resin, cycloaliphatic resin and mixtures thereof.

2.   The pressure sensitive adhesive composition of claim 1, wherein the composition further comprises vitamin A and vitamin E.

3.   The pressure sensitive adhesive composition of claim 2, further comprising at least one hydrophilic liquid absorber or gel thickener.

4.   The pressure sensitive adhesive composition of claim 3, wherein the hydrophilic liquid absorber or gel thickener is selected from the group consisting of guar gum, locust bean gum, pectin, alginates, gelatin, xanthum, gum karaya, sodium carboxymethylcellulose, methylcellulose, hydroxypropylmethylcellulose, sodium starch glycolate, polyvinyl alcohol, polyethylene glycol, and any combination of two or more of the hydrophilic liquid absorbers or gel thickeners.

5.   The pressure sensitive adhesive composition of claim 1, wherein the composition has an elastic modulus of less than 1000000 Pa at 1 Hz.

6.   The pressure sensitive adhesive composition of claim 2, wherein the composition has an elastic modulus of less than 300000 Pa at 1 Hz.

7.   The pressure sensitive adhesive composition of claim 1, wherein the homopolymer or copolymer is selected from the group consisting of ethylene vinyl acetate carbon monoxide and ethylene butyl acrylate carbon monoxide.

8.   The pressure sensitive adhesive composition of claim 1, wherein the homopolymer or copolymer is selected from the group consisting of ethylene butyl acetate, ethylene vinyl alcohol, and ethylene butyl acrylate.

9.   The pressure sensitive adhesive composition of claim 1, wherein the composition has a tan ($\delta$) of at least 0.8 at 1 Hz.

10.   The pressure sensitive adhesive composition of claim 9, wherein the composition has a tan ($\delta$) of at least 1.0 at 1 Hz.

**Patentansprüche**

1.   Haftklebstoffzusammensetzung, umfassend:

a. ein Homopolymer oder Copolymer mit einem Gewichtsanteil von 2 bis 45 %, zusammengesetzt aus Polyisobutylen, Ethylenvinylacetat, Ethylenvinylacetat-Kohlenmonoxid, Ethylenbutylacetat, Ethylenvinylalkohol, Ethylenbutylacrylat, Ethylenbutylacrylat-Kohlenmonoxid, Polystyrolcopolymeren und Mischungen davon;
b. Sheabutter mit einem Gewichtsanteil von 1 bis 10 %;
c. einen Klebrigmacher mit einem Gewichtsanteil von 15 bis 45 %, zusammengesetzt aus natürlichem Kolophonium, modifiziertem Kolophonium, Glycerolester von natürlichem Kolophonium, Glycerolester von modifiziertem Kolophonium, Pentaerythritolester von natürlichem Kolophonium, Pentaerythritolester von modifiziertem Kolophonium, phenolmodifiziertem Terpenharz, aliphatischem Erdölkohlenwasserstoffharz, zykloaliphatischem Harz und Mischungen davon.

2. Haftklebstoffzusammensetzung gemäß Anspruch 1, wobei die Zusammensetzung ferner Vitamin A und Vitamin E umfasst.

3. Haftklebstoffzusammensetzung gemäß Anspruch 2, ferner umfassend mindestens einen hydrophilen Flüssigkeitsabsorber oder ein Gelverdickungsmittel.

4. Haftklebstoffzusammensetzung gemäß Anspruch 3, wobei der hydrophile Flüssigkeitsabsorber oder das Gelverdickungsmittel zusammengesetzt sind aus Guargummi, Johannisbrotgummi, Pektin, Alginaten, Gelatine, Xanthan, Karayagummi, Natrium-Carboxymethylcellulose, Methylcellulose, Hydroxypropylmethylcellulose Natriumstärkeglycolat, Polyvinylalkohol, Polyethylenglycol und jede Kombination von zwei oder mehr der hydrophilen Flüssigkeitsabsorbern oder Gelverdickungsmitteln.

5. Haftklebstoffzusammensetzung gemäß Anspruch 1, wobei die Zusammensetzung bei 1 Hz einen Elastizitätsmodul von unter 1000000 Pa aufweist.

6. Haftklebstoffzusammensetzung gemäß Anspruch 2, wobei die Zusammensetzung bei 1 Hz einen Elastizitätsmodul von unter 300000 Pa aufweist.

7. Haftklebstoffzusammensetzung gemäß Anspruch 1, wobei das Homopolymer oder Copolymer zusammengesetzt sind aus Ethylenvinylacetat-Kohlenmonoxid und Ethylenbutylacrylat-Kohlenmonoxid.

8. Haftklebstoffzusammensetzung gemäß Anspruch 1, wobei das Homopolymer oder Copolymer zusammengesetzt sind aus Ethylenbutylacrylat, Ethylenvinylalkohol und Ethylenbutylacrylat.

9. Haftklebstoffzusammensetzung gemäß Anspruch 1, wobei die Zusammensetzung bei 1 Hz einen Verlustfaktor ($\delta$) von mindestens 0,8 aufweist.

10. Haftklebstoffzusammensetzung gemäß Anspruch 9, wobei die Zusammensetzung bei 1 Hz einen Verlustfaktor ($\delta$) von mindestens 1,0 aufweist.

**Revendications**

1. Une composition adhésive sensible à la pression comprenant :

   a. 2 à 45 % en poids d'un homopolymère ou copolymère choisi dans le groupe constitué par le polyisobutylène, l'éthylène-acétate de vinyle, l'éthylène-acétate de vinyle monoxyde de carbone, l'éthylène-butylacétate, l'éthylène-alcool vinylique, l'éthylène-butylacrylate, l'éthylène-butylacrylate monoxyde de carbone, les copolymères de polystyrène et leurs mélanges,
   b. 1 à 10 % en poids de beurre de karité ;
   c. 15-45 % en poids d'une résine tackifiante choisi dans le groupe consistant en colophane naturelle, colophane modifiée, ester de glycérol de colophane naturelle, glycérol-ester de colophane modifiée, ester pentaérythritol de résine naturelle, ester pentaérythritol de colophane modifiée, phénolique de terpène de résine modifié, la résine hydrocarbonée aliphatique de pétrole, la résine cycloaliphatique et leurs mélanges.

2. La composition adhésive sensible à la pression selon la revendication 1, dans laquelle la composition comprend en outre la vitamine A et la vitamine E.

3. La composition adhésive sensible à la pression selon la revendication 2, comprenant en outre au moins un absorbant de liquide hydrophile ou un épaississant de gel.

4. La composition adhésive sensible à la pression selon la revendication 3, dans laquelle l'absorbant de liquide hydrophile ou l'épaississeur de gel est choisi parmi le groupe constitué de la gomme de guar, la gomme de caroube, la pectine, les alginates, la gélatine, le xanthane, la gomme karaya, la carboxyméthyl-cellulose sodique, la méthylcellulose, l'hydroxypropylméthylcellulose, le glycolate d'amidon sodique, l'alcool polyvinylique, le polyéthylèneglycol et toute combinaison de deux ou plus des absorbants de liquides hydrophiles ou des épaississants de gel.

5. La composition adhésive sensible à la pression selon la revendication 1, dans laquelle la composition a un module d'élasticité inférieur à 1 000 000 Pa à 1 Hz.

6. La composition adhésive sensible à la pression selon la revendication 2, dans laquelle la composition a un module d'élasticité inférieur à 300 000 Pa à 1 Hz.

7. La composition adhésive sensible à la pression de la revendication 1, dans laquelle l'homopolymère ou le copolymère est dérivé du groupe comprenant l'éthylène-acétate de vinyle monoxyde de carbone et l'éthylène butylacrylate monoxyde de carbone.

8. La composition adhésive sensible à la pression de la revendication 1, dans laquelle l'homopolymère ou le copolymère est choisi parmi le groupe constitué de l'éthylène butylacrylate, éthylène-alcool vinylique, et l'éthylène butylacrylate.

9. La composition adhésive sensible à la pression de la revendication 1, dans laquelle la composition a un tan ($\delta$) d'au moins 0,8 à 1 Hz.

10. La composition adhésive sensible à la pression de la revendication 9, dans laquelle la composition a un tan ($\delta$) d'au moins 1.0 à 1 Hz.

Figure 1

Figure 2

**REFERENCES CITED IN THE DESCRIPTION**

**Patent documents cited in the description**

- US 6437038 B
- US 4551490 A
- US 6933342 B
- US 6685683 B
- US 3712304 A
- US 3799166 A
- US 5633010 A
- US 20110251300 A
- US 20100204632 A
- US 20110251542 A
- WO 2009006901 A
- US 20110118363 A
- US 20040241246 A
- US 20110251542 A1
- US A1 A
- US 20100204632 A1